# EUROPEAN PATENT APPLICATION

(11) **EP 0 549 341 A1**
(43) Date of publication of application: **30.06.1993**
(21) Application number: 92311756.8
(22) Date of filing: 23.12.1992
(51) Int. Cl.: A61M 1/14, A61M 1/34, A61B 5/14

(54) **Hollow fiber plasma sampler**

(30) Priority: 24.12.1991 US 816427
(71) Applicant: W.R. Grace & Co.-Conn., New York, New York 10036 (US)
(72) Inventor: Patti, Kenneth J., Massachusetts 01845 (US); Fecondini, Luciano, I-40126 Bologna (IT); Cekala, Chester, Massachusetts 02193 (US)
(74) Representative: Barlow, Roy James

(57) **Abstract**

Exemplary methods and systems of the invention involve the use of a high performance capillary filter sampler (10) to separate a permeate fluid for sampling without requiring first that blood cells be separated such as by centrifugation. The invention affords particular advantages in performing continuous, extracorporeal sampling and monitoring of blood in situations requiring minimal exposure to infection risks and minimal depletion of blood cells.

## Description

### FIELD OF THE INVENTION

The present invention relates to the field of liquid purification and separation, and more particularly to filtration of biological fluids such as blood.

### BACKGROUND OF THE INVENTION

The practice of sampling and testing blood and other bodily fluids entails potential contamination or infection by bacteria, viruses such as hepatitis, and other bloodborne diseases. Blood is typically withdrawn from the patient percutaneously or indirectly from a "T-type" valve installed in a blood tubing circuit connected to the patient. The sample is then placed into a test tube. In most instances, blood serum is separated from blood cells by centrifugation or coagulation. The serum then must be removed from the sedimented or clotted blood cells, which are discarded, and transferred by pipette to another container or vessel for analysis. The risks of infection, contamination, breakage of sample vial, or of aerosoling the sample increase with the number of times that the sample must be handled or transferred.

Sampling and testing of blood serum, plasma water, and their constituents, especially when performed on a continuous basis, depletes a relatively large cumulative sample volume. This may be the case in continuous monitoring of blood sugar levels or electrolyte levels, or in renal studies of urea and creatinine levels. In certain cases, such as patients who are anemic or who are undergoing chronic hemodialysis, which results in a drop in normal blood cell count, the further reduction of blood cells may be contraindicated.

In view of the alarming need to minimize exposure of health care staff to virally transmitted diseases, and specifically in the area of AIDS prevention, there is an increased need for systems and methods for sampling and monitoring blood fluids which minimize sampling volumes and frequencies.

### SUMMARY OF THE INVENTION

The invention provides a system and method which relies upon the use of a hollow fiber filter sampler for separating a sample fluid that permeates from intercapillary to extracapillary space. The invention has advantageous features especially when employed on a continuous basis, such as filtration/sample collecting and monitoring of biological fluids and fluid systems and, in particular, the blood circulatory system of humans and animals. The invention provides advantages over conventional blood sampling and monitoring methods because it avoids unnecessary steps such as centrifugation or coagulation of cells, thereby conserving patient blood cells and blood volume, decreasing the potential risks of infection to the handler, and preventing bacterial contamination to the sample.

Exemplary systems and methods described herein involve a drastically miniaturized on-line filtration sampler comprising at least one semi-permeable hollow-fiber filter capillary contained in a flow-through casing for separating a sample permeate from intercapillary space. The sample permeate may be sampled in the extracapillary space, intermittently or on a continuous basis, or removed therefrom for intermittent or continuous sampling. Preferably, the sampler contains a parallel plurality of densely-packed, high performance, hollow-fiber capillaries, and a flow-through casing which separates intercapillary flow-through spaces from the extracapillary space into which the sample fluid permeates.

Further exemplary embodiments involve the use of a sampling port with tubing connected to the extracapillary space, and a governor for limiting the rate of sample withdrawal. In other exemplary embodiments, the extracapillary space within the sampler has minimal volume to avoid dilution and stagnation of sample fluid.

Further exemplary systems involve the use of a pump to remove fluid permeate sample from the sampler to an autoanalyzer or other sampling/monitoring device on a continuous basis. The sampling/monitoring device can be used to control other mechanisms, such as blood or dialyzer mechanisms, within the extracorporeal blood circuit.

The sampling method of the invention comprises the step of providing a sampler having at least one flow-through capillary and a case thereabout operative to define intracapillary space for flow-through of fluid within said circuit and extracapillary space for receiving fluid sample which permeates through said capillary from said intracapillary space. Further exemplary methods comprise the steps of sampling the fluid permeate in the extracapillary space, or withdrawing it for sampling. Further exemplary methods involve feeding the permeate sample continuously or periodically to an autoanalyzer. The autoanalyzer may be preprogramed to provide an output when a certain condition is detected. The output may be used to provide an alarm, to adjust the speed of blood pumps, dialysate pumps, drain pumps, and other pumps, and to control other blood circuit mechanisms or operations.

### DESCRIPTION OF THE DRAWINGS

The advantages and features of the method and system of the invention will be more readily understood when the following detailed description is considered in conjunction with the drawings, wherein:
Fig. 1 is a side view of an exemplary filtration sampler of the invention which may be used in exemplary sampling and monitoring methods and systems of the invention;
Fig. 2 is a view along A of the sampler shown in Fig. 1;
Fig. 3 is a schematic representation of an extracorporeal blood circuit and a schematic diagram of an exemplary sampling device and method of the invention in conjunction therewith, and;
Fig. 4 is a schematic representation of an extracorporeal blood circuit and a schematic diagram of an exemplary sampling/monitoring system and method of the invention wherein sample is fed continuously or periodically from the blood circuit to a sampling and/or monitoring device.

### DETAILED DESCRIPTION OF THE INVENTION

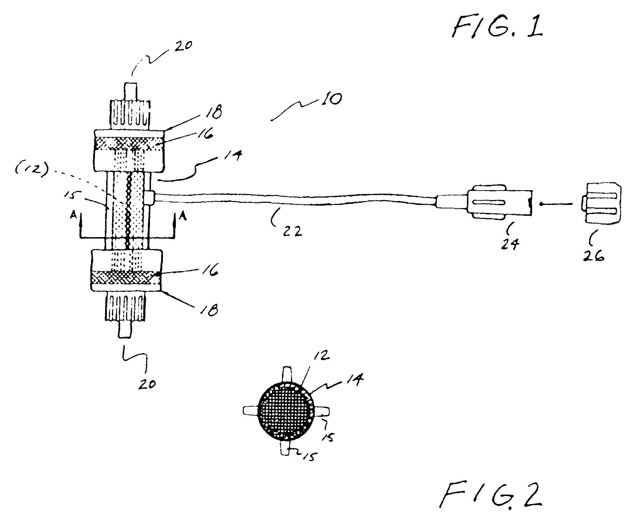

Fig. 1 shows an exemplary fluid sampling system 10 of the present invention which is comprised of a bundle (shown by dotted lines) of hollow-fiber capillaries (12) or filter tubules surrounded by a case 14 whereby an intercapillary space and extracapillary space are defined and separated from each other. Ends of the capillaries are tightly packed in a preferably dense, parallel arrangement and potted in a layer of sealant material 16 such as polyurethane. The sealed bundle is then cut or sliced orthogonally to expose open capillaries (12) at each end. The bundle of capillaries (12) with sealant 16 is packed into the case 14 and capped at either end by cap members 18 which may contain twist-on blood inlet and outlet ports 20 or any connector compatible with blood tubing.

Intercapillary spaces are physically separated from extracapillary spaces within the case, communicating only through the selectively permeable capillary 12 wall membrane. Blood from the tubing of an extracorporeal blood circuit flows into the sampling system 10 through a blood inlet port 20, through intercapillary spaces, across the membranes (12), out of the sampling system 10 through a blood outlet port 20, and returned to the extracorporeal blood circuit.

Fig. 2 shows a representational cross-sectional view along A of the exemplary capillary bundle arrangement 12, case 14, and longitudinal case reinforcement members 15 shown in Fig. 1. Fig. 1 and 2 are for illustrative purposes only. Preferably, the bundled capillaries 12 are densely packed within the casing 14 such that almost no space can be seen between the capillaries, which will look unordered and "smashed" together when actually packed within the casing 14, and almost no space can be seen between the capillaries 12 and the wall of the casing 14.

Capillaries 12 or tubule shaped filters in an exemplary embodiment of the invention may be comprised of hollow fibers of the type used for ultrafiltration (UF) or microfiltration (MF). UF refers typically to the filtration of molecules ranging between 10,000 and 1,000,000 in molecular weight, while MF typically refers to filtration of molecules exceeding this range. The pore sizes in capillaries typically ranges between 10 and 1,000 Angstroms for UF and between 1,000 and 100,000 Angstroms for NF (1 micron = 10,000 Angstroms). A variety of pore sizes and filtration ranges can be used within contemplation of the invention.

In an exemplary embodiment of the invention, the capillaries are made of hollow-fiber ultrafilters (UF) made of polysulfone, consisting of a very thin (0.2 - 0.45 micrometers) dense "skin" of extremely fine, controlled pore structure to prevent permeation by blood cells and/or other blood constituents which are not desired in the fluid permeate to be sampled. The "skin" is preferably disposed on the inward surface of the capillary. For continuous monitoring purposes described hereinafter, the sampling rate across the membrane is preferably about five to six drops per minute when the sampling device 10 is placed within one foot below the level of the patient. Other known capillary fibers, fiber materials, and hollow fiber filters may be used within contemplation of the invention. For example, ENKE polypropylene fibers, which have 0.2 micron pore size, have no skin, and are isotropic, are also believed to be suitable when used within contemplation of the invention.

An exemplary sampler of the invention contrasts known hemofilters used in hemodiafiltration. Current high performance hemofilters typically contain thousands of bundled capillaries and permit dialysate to be circulated within the capillary chamber for maximum clearance exchange of solute constituents across the membrane and, hence, large volume flow capabilities in the extracapillary space. In hemodialysis, solute moves from blood to dialysate by diffusing down a concentration gradient. Solute removal from blood is proportional to membrane-surface area and to the flow rates of blood and dialysate. Accordingly, hemofilters currently available have large membrane-surface areas requiring proportionally large cases and, consequently, having proportionally large intercapillary and extracapillary spaces. Moreover, hemofilters which are commercially available and currently known contain two or more ports connected to the extracapillary chamber (e.g., for ultrafiltrate inlet and outlet), each of which contains ports in which ultrafiltrate fluid can become trapped and stagnant and which otherwise permit a large dilutionary volume of ultrafiltrate or permeate fluid to accumulate. The typical flow rates through the ports of such commercially available hemofilters ranges from 100 to 500 ml./min.

In contrast, an exemplary sampler 10 of the invention has internal dimensions and volume more than one hundred times smaller than conventional hemofilters. In other exemplary samplers of the invention, the extracapillary chamber is of minimal volume (e.g., all capillaries are tightly packed against each other and within the sampler such that the capillaries physically contact each other and the inner walls of the sampler case 14) so as to minimize dilutionary and stagnatory effects upon the fluid which permeates into the extracapillary space. This facilitates accurate and efficient sampling of blood flowing through the intercapillary passages 16 of the sampling device 10 because dilutionary and stagnatory effects caused by overly large extracapillary spaces are decreased, and the sample taken is more likely to be an accurate representation of the fluid passing through the intercapillary space at any given time. The sampler facilities priming because the sampler can be filled with fluid more quickly because it requires less fluid. In a preferred embodiment, the internal dimensions of the casing, in other words, the cylinder of space in which the capillaries 12 are bundled between the sealant material 16 of the end caps and within the generally cylindrical casing 14, measures approximately 4-6 mm. diameter by 15-30 mm. length.

In further exemplary embodiments, fluid which permeates into the extracapillary space may be withdrawn therefrom through a removal outlet, valve, or preferably a tube 22 which has a gasket port 24 adapted for hypodermic needles. The tube 22 is preferably smaller in diameter than standard tubing customarily used for intravenous (IV) infusion or dialysis. The port 24 can be conformed to receive a port cap 26 for further protection against contamination and infection. The withdrawal tube 22 is preferably comprised of flexible material having a relatively small inner diameter, preferably less than one-eighth inch. More preferably, the tube 22 has an inner diameter or point of narrowing which is predetermined in dimension so as to limit or govern the rate at which sample is withdrawn, thereby preventing the open pore filter membranes 12 from being clogged by cells or other blood constituents.

In further exemplary sampling devices a valve or stopcock instead of tubing permits fluid to be withdrawn, such as by needleless syringe or pump bulb, and deposited directly into an analyzer or testing device.

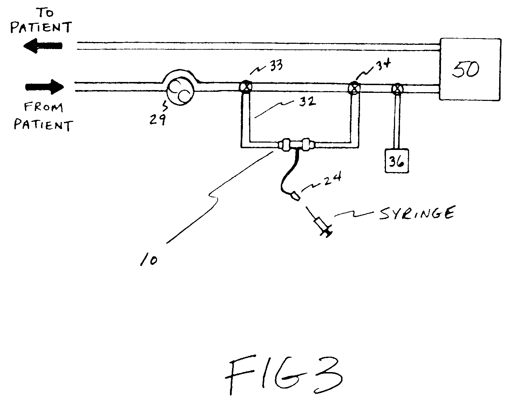

Fig. 3 is a schematic representation of the sampler 10 of Figs. 1 and 2 used in an exemplary sampling/monitoring system installed in an extracorporeal dialysis circuit. A hollow fiber sampler device 10 may be placed anywhere on the extracorporeal blood circuit, but is preferably placed within a parallel shunt 32 connected by T-connectors or three-way valves 33 and 34 within the blood circuit at a point subsequent to (downstream from) a blood pump 29 typically used to move blood from the patient. The shunt 32 is operative to permit blood to flow through the sampler system 10 without substantially impeding the flow of blood through the extracorporeal circuit. The sampler 10 is also preferably located prior to (upstream from) a heparin infusion pump 36 within the extracorporeal blood circuit and prior to any hemodialysis or hemodiafiltration device 50. The device 50 may be a known dialysis, hemodialysis, or hemodiafiltration device, or any other known device or system used for altering or adjusting or filtering blood components and fluids on a continuous or intermittent/batchwise basis.

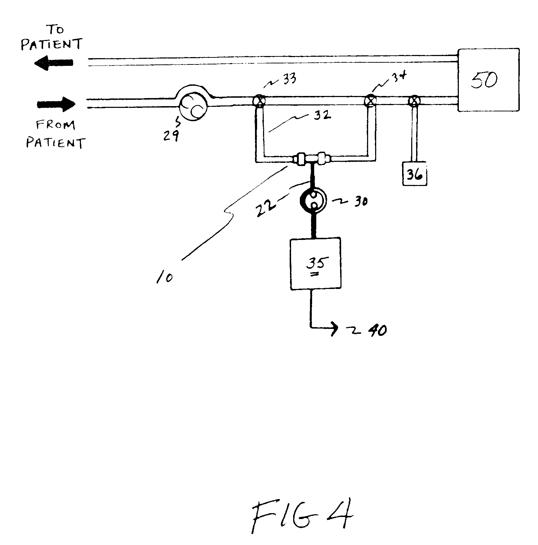

Fig. 4 is a schematic representation of another exemplary blood sampling/monitoring system of the invention which comprises a blood sampler 10, tubing 22 or piping for removing sample fluid from the extracapillary space of the sampler 10, a pump 30 or other device for pumping the sample fluid at a steady or intermittent controllable rate to a sampler device 35, such as an autoanalayzer, electrolyte monitoring device, toxicity monitoring device, or other known sampling and/or monitoring devices for any number of collection, sampling, monitoring, fluid or electrolyte level adjustment, and/or feedback purposes. In further exemplary embodiments, the sampling and monitoring device 35 detects the level of a particular constituent in the sample and sends an output signal 40 to an alarm (not shown) for indicating the attainment of a predetermined threshold or presence of a dangerous condition. A signal 40 can be sent by the device 35 to any number of devices or mechanisms connected to the dialysis or dialfiltration machine 50, such as to blood pumps, dialysate pumps, drain pumps, or other mechanisms. One signal output 40 or several signal outputs can be used for individual or assorted control and feedback purposes.

An exemplary method for sampling fluids comprises the steps of providing a hollow fiber filter device, of the type shown in Fig. 1 and 3, and permitting sample fluid to permeate into the extracapillary space of the sampler 10, thereby separating serum from the blood stream without having to coagulate or centrifuge the sample or to remove large amounts of blood volume from the patient or extracorporeal blood circuit. Further exemplary methods involve sampling the fluid permeate within the sampler case 14, such as by installing sensors or monitor devices within the case or by withdrawing fluid permeate from the extracapillary space, such as by syringe as shown in Fig. 3, for evaluation on a continuous and/or intermittent basis. Sampling or monitoring may thus be performed without having to centrifuge or coagulate blood or remove large cumulative blood volume from the extracorporeal blood circuit.

A further exemplary method of sampling/monitoring fluid in an extracorporeal circuit comprises the steps of providing a sampler having at least one hollow fiber filter 10 and sampling the permeate fluid in the extracapillary space, or withdrawing fluid therefrom, for sampling and/or monitoring on a continuous or intermittent basis. Further exemplary methods involve using a pump 30 and an autoanalyzer electrolyte monitor, or other sampling and/or monitoring devices 35. Samples may be withdrawn through a valve or self-sealing needle gasket device which is installed along the tubing 22 or piping connected to the sampler on a continuous basis. The tubing or piping 22 should preferably be as short as possible to minimize the amount of serum or plasma water removed from the extracorporeal circuit. The serum or plasma water removed for collection, sampling, and/or monitoring may be returned to the extracorporeal circuit, if desired, by any known means, such as by tubing (not shown) connected downstream of the sampler 10 or connector 34.

The method, sampler, and system of the invention are particularly useful in the medical field for dialysis, toxicology studies, open-heart surgery, and similar uses. The method, sampler, and system of invention can also be useful in a fermenting tester and in bioreactors. As modifications and variations of the methods of the invention may be evident to those skilled in the art, the scope of the invention is limited only by the scope of the claims.

## Claims

1. A method for sampling fluid comprising the step of providing a sampler device having at least one flow-through capillary, preferably an ultrafilter comprised of polysulphone, and a casing thereabout operative to define intracapillary space for flow-through of fluid and extracapillary space for receiving fluid sample which permeates through said capillary from said intracapillary space, and thereafter analysing said permeated fluid sample.

2. The method of claim 1, further comprising the step of providing a circuit, preferably an extracorporeal blood circuit, more preferably including a dialysis machine, in which biological fluid may travel.

3. The method of claim 1, further comprising the step of providing said sampler within an extracorporeal blood circuit downstream from a blood pump or a heparin infusion pump.

4. The method of claim 2 or 3, further comprising the steps of providing in said extracorporeal circuit a hollow fibre haemofilter operative for continuous haemofiltration, preferably operative for continuous haemodiafiltration and infusion of dialysate.

5. The method of any one of claims 2 to 4, comprising providing said sampler in a parallel shunt circuit connected to said extracorporeal blood circuit, said shunt circuit being operative to permit blood to flow through said sampler without substantially restricting blood flow through said circuit.

6. The method of any one of the preceding claims, further comprising the step of removing fluid which has permeated from said intracapillary space to said extracapillary space, preferably by removing permeate fluid from said extracapillary space for analysing.

7. The method of any one of the preceding claims, wherein the sampler device has a plurality of capillaries disposed in a generally parallel arrangement, said capillaries being further packed tightly together within and against said casing such that minimum extracapillary space is achieved and whereby dilution and stagnation of fluid that permeates into said extracapillary space is minimised.

8. The method of any one of claims 1 to 6, wherein said sampler device comprises a plurality of capillaries permeable to blood serum and impermeable to blood cells.

9. The method of any one of claims 1 to 8, further comprising the step of providing a limiter, optionally integrally connected to said sampler, operative to limit the rate at which sample fluid is withdrawn from said casing, whereby the tendency for blood cells to clog said capillary, when permeable sample is withdrawn from said extracapillary space, is decreased.

10. The method of claim 1, wherein said sampler device further comprises a tubing connected to said extracapillary space within said casing, said tubing having a predetermined inner dimension whereby the rate of sample withdrawal from said casing is governed.

11. The method of any one of claims 1 to 9, wherein said sampler device further comprises tubing, preferably having a diameter smaller than standard intravenous tubing, and means for receiving a needle operative to withdraw a sample from said sampling device.

12. The method of any one of claims 1 to 9, further comprising the steps of:
providing tubing operative to permit withdrawing sample fluid from said extracapillary space defined within said casing; and
connecting to said withdrawal tubing an automatic sampling device, preferably including a pump along said tubing for withdrawing fluid, said withdrawal tube and sampling device being operative to provide continuous monitoring of fluid within said circuit.

13. The method of any one of claims 1 to 9, further comprising the steps of:
providing tubing operative to permit withdrawal of sample fluid from said extracapillary space defined within said casing; and
connecting an automatic sampling device to said withdrawal tubing, said sampling device being operative to sense a condition in the sample fluid and to provide an output, preferably an audible alarm, corresponding to said sensed condition.

14. A dialysis system comprising:
tubing connected to a patient circulatory system;
a dialysis device connected to said blood tubing; and
a sampler connected to said blood tubing, said sampler having at least one flow-through capillary and a case thereabout operative to define intracapillary space for flow-through of fluid within said circuit and extracapillary space for receiving fluid which permeates through said capillary from said space.

15. The system of claim 13, further comprising a means for permitting withdrawal of permeate fluid sample from said extracapillary space.

16. The system of claim 13, further comprising a device for automatically sampling permeate fluid in said extracapillary space and providing an output in accordance with the sampling.

17. A fermenting tester system, comprising:
a fermenter; and
a sampler connected to said fermenter and operative for testing fluid in said fermenter, said sampler having at least one flow-through capillary and a casing thereabout operative to define intracapillary space for flow-through of fluid within said circuit and extracapillary space for receiving fluid which permeates through said capillary from said space, said permeated fluid providing a sample.

18. A fluid sampler, comprising:
at least one capillary selectively permeable to the fluid desired to be sampled;
a casing disposed thereabout and operative to define intracapillary space for flow-through of fluid and extracapillary space operative to receive fluid which permeates through said capillary from said intracapillary space; and
a limiter operative to govern the rate at which permeated sampler can be withdrawn from the extracapillary space.
